# EUROPEAN PATENT APPLICATION

(11) **EP 1 486 116 A1**
(43) Date of publication of application: **15.12.2004**
(21) Application number: 03707150.3
(22) Date of filing: 27.02.2003
(51) Int. Cl.: A01K 67/027, C12N 5/06

(54) **METHOD OF CONSTRUCTING GERMLINE CHIMERIC ANIMAL**

(30) Priority: 27.02.2002 JP 2002051303
(71) Applicant: Nagao, Yasumitsu, Kyoto-shi, Kyoto 606-8237 (JP); Imai, Hiroshi, Otsu-shi, Shiga 520-0014 (JP); Horii, Takuro, Maebashi-shi, Gunma 371-0031 (JP); Totsuka, Yoshikazu, Utsunomiya-shi, Tochigi 320-0065 (JP)
(72) Inventor: Nagao, Yasumitsu, Kyoto-shi, Kyoto 606-8237 (JP); Imai, Hiroshi, Otsu-shi, Shiga 520-0014 (JP); Horii, Takuro, Maebashi-shi, Gunma 371-0031 (JP); Totsuka, Yoshikazu, Utsunomiya-shi, Tochigi 320-0065 (JP)
(74) Representative: Denison, Christopher Marcus
(86) International application number: PCT/JP2003/002266
(87) International publication number: WO 2003/071869

(57) **Abstract**

The present invention provides a method for efficiently producing a germ-line chimeric animal and a method for conveniently producing a heterozygous animal and a homozygous animal. Specifically, a chimeric animal wherein germ cells are derived from introduced ES cells is produced by injecting embryonic stem cells (ES cells) into an early embryo (blastocyst-stage embryo) incapable of forming germ cells because of a genetic factor. A homozygote is obtained by crossing a female heterozygote with a male heterozygote obtained from the obtained chimeric animals. The thus obtained chimeric animal is extremely useful for conducting gene function analyses and the like.

## Description

### [TECHNICAL FIELD]

The present invention relates to a method for producing a chimeric animal wherein germ cells are formed, using an early embryo (generally, a blastocyst) genetically incapable of forming germ cells and totipotent cells, and a chimeric animal individual that is obtained by this method and wherein the germ cells are derived from totipotent cells. Totipotent cells used herein are pluripotent cells capable of differentiating into germ cells. Furthermore, the present invention relates to a heterozygous animal and a homozygous animal obtained from the chimeric animal.

### [BACKGROUND ART]

A technique of returning embryonic stem cells (ES cells) into embryos to cause ontogenesis (Evans MJ & Kaufman MK, Nature 292, 154-156 (1981)) and a technique of causing homologous recombination in ES cells (Zijlstra M et al., Nature 342, 435-438 (1989); Thompson S et al., Cell 56, 313-321 (1989)) have been established as a result of progress in development engineering technology. These techniques have been combined using mice for the first time, thereby establishing technology for producing gene-altered mice such as knockout mice. The thus completed technology enables the production of mutants and then the investigation of gene functions using mammals, which could have been conducted using only yeast or Drosophila. This is attracting attention as an important technology essential for gene function analyses in the post-genome age.

Regarding the efficient production of gene-altered animals such as knockout mice, there are 3 main points. The 1 st point involves the establishment of good ES cells. The 2^{nd} point involves the efficient alteration of genes in ES cells. The 3^{rd} point is that the gene-altered ES cells forms a chimeric mouse and in the obtained chimeric mouse, gene alteration conducted in the ES cells are transmitted to a germ line so as to be transmitted to the next generation.

Among technologies that have been developed thus far, the 1^{st} point, establishment of good ES cells, depends largely on a mouse strain. In addition to low establishment probability, successfully established ES cells lose their ability to be transmitted to germ line as subculture is repeated, so that ES cells established as a result of much effort are currently used without repeating subculture to as great an extent as is possible. This tendency is particularly significant when ES cells of an inbred mouse are used.

Regarding the 2^{nd} point, homologous recombination technology and related technologies have been improved repeatedly, and they are frequently utilized as technologies at the stage of practical application.

The 3^{rd} point involves many problems. In particular, many researchers have gone through a bitter experience such as learning that chimeric mice could be produced, but they showed no germ-line transmission. In knockout mouse production, the 3^{rd} point represents a major obstacle. Hence, a currently employed actual method involves obtaining and using ES cells proved to be transmitted to germ line and subcultured in as small a quantity as is possible.

In the meantime, ES cells are thought to be capable of achieving normal development in all cell lineages composing a conceptus, but to have low capability of contributing an exocelomic cell lineage, such as that of a placenta, compared with an embryo (generally, a blastocyst) into which ES cells are injected in the ontogeny process (Beddington R S & Robertson E J, Development 105, 733-737 (1989); Nagy A et al., Development 110, 815-821 (1990)). Hence, a technique that has been proposed for obtaining a mouse wherein all tissues including the genital tissue are derived from ES cells involves combining ES cells and an embryo whose development and differentiation orientations are mainly biased to an exocelomic placenta, and thus introducing the ES cells into development and differentiation orientation towards the embryo. In this case, a combination of mouse ES cells and a mouse tetraploid embryo is thought to be the most appropriate combination. Specifically, a tetraploid embryo alone rarely develops after embryo implantation, and almost none of the embryos reach the second trimester (Kaufman M H & Webb S, Development 110, 1121-1132 (1990)). It has been reported that in the case of a chimera produced from a tetraploid embryo and a normal embryo (diploid), the tetraploid cell hardly ever contributes to the embryo itself. However, it contributes to a considerable extent to the exocelomic membrane tissue (Tarkowski A K, J. Embryol. Exp. Morph. 41, 47-64 (1977)). Thus, a method that has been proposed involves combining mouse ES cells with a mouse tetraploid embryo so that they complement to each other, so as to produce an associated chimera. In this case, it has been reported that polar chimeras are produced wherein fetuses are derived from the ES cells. However, most exocelomic tissues are derived from the tetraploid cell (Nagy A et al., Development 110, 815-821 (1990)). However, successful examples of obtaining chimeric individuals from ES cells of inbred mice by this technique are extremely rare. Even in a case of successfully obtaining such chimeric individuals, the chimeric mice have been unable to survive long because of developing disorders such as respiratory disturbances or malformation (Eggan K et al., PNAS 98, 6209-6214 (2001)). Hence, it has been difficult to use this method as a practical method for producing a germ line chimera.

Accordingly, it is earnestly desired to develop technology for forming gene-altered chimeric individuals, and for efficiently producing next-generation individuals wherein gene alteration derived from ES cells in the thus obtained chimeric individuals can be transmitted to a germ line.

### [SUMMARY OF THE INVENTION]

In view of the above circumstances, we have intensively studied the methods for producing a germ-line chimera. Specifically, an object to be achieved or a purpose of the present invention is to efficiently produce a gene-altered animal such as a knockout mouse using totipotent cells such as ES cells that have been subjected to gene alteration, and particularly, to construct a method for efficiently producing a germ-line chimeric mouse. Specifically, in the production of a gene-altered mouse such as a knockout mouse, researchers have experienced a problem in that a chimeric mouse can be produced using gene-altered ES cells, although the gene-altered ES cells are unable to be transmitted to a germ line in the obtained chimeric mouse, so that gene alteration is unable to be transmitted to the next generation. Thus, production of chimeric mice from which offspring having an altered gene can be obtained has been desired, and the problem must be addressed. The present invention has been achieved to address such problem of a lack of transmission of such altered gene to the next generation.

Accordingly, we have conceived of the production of a germ-line chimeric animal derived from totipotent cells using an early embryo incapable of forming germ cells and the totipotent cells. Thus, we have completed the present invention. Examples of totipotent cells used herein include pluripotent cells capable of differentiating into germ cells, and specifically, stem cells such as ES cells and EG cells, primordial germ cells, inner cell mass, and reconstructed embryos such as nuclear transferred embryos.

An object of the present invention is to provide a method for producing a germ-line chimeric animal derived from totipotent cells by introducing totipotent cells such as ES cells into an early embryo incapable of forming germ cells, and to provide an animal wherein gene alteration conducted for ES cells or the like is transmitted to offspring.

Hence, we have studied a method for efficiently producing a germ-line chimeric mouse using totipotent cells such as ES cells (embryonic stem cells) to achieve the aforementioned object.

A germ-line chimera in the present invention means a case where germ cells of a chimeric animal are derived from the totipotent cells introduced. In general, whether or not a germ-line chimeric mouse produced using ES cells is a germ-line chimeric mouse cannot be confirmed before these steps of: producing a chimeric mouse between a line from which the ES cells have been derived and a line having different coat color; and obtaining pups by crossing the obtained chimeric mouse with the mouse of the line from which the ES cells have been derived, or with the mouse of the line having different coat color, so as to confirm the coat color of the line from which the ES cells have been derived. Particularly in the case of male animals, even if a germ cell is formed from ES cells, it is difficult to obtain pup having the gene of the ES cells when the contribution rate of the ES cells to the germ cell is low. Furthermore, when the number of sperm cells derived from ES cells is low or sperm activity is low, fertilization cannot be achieved and pups cannot be obtained. Furthermore, when gene alteration is conducted for mitochondria, since the mitochondria are derived from oocytes, female ES cells must be used to produce female germ-line chimeric mice.

If chimeric mice having only germ cells derived from ES cells within testes or within ovaria can be obtained, pups can certainly have chromosomes derived from the ES cells, so that germ-line chimeric mice can be efficiently produced. Even when male chimeric mice are obtained, but the number of sperm cells of the obtained chimeric mice is low, or sperm activity is low, pups can be easily obtained using technology such as intracytoplasmic sperm injection (ICSI).

In the case of female chimeric mice, pups can be obtained by natural mating, artificial insemination, in vitro fertilization, or intracytoplasmic sperm injection.

The present invention has been achieved based on the above studies and relates to a method for producing a germ-line chimeric animal. Furthermore, the present invention relates to a chimeric animal obtained by this method and a gene-altered animal obtained from the chimeric animal. Specifically, the present invention is a method for producing a chimeric animal wherein germ cells are derived from totipotent cells using an early embryo (generally, a blastocyst) incapable of genetically forming germ cells and the totipotent cells, and a germ-line chimeric animal obtained by this method. An early embryo incapable of genetically forming germ cells in the present invention can be produced utilizing a genetic factor whereby an inter-specific F1 hybrid lacks germ cells. In which cells (that is, female germ cells or male germ cells) the germ cell deficiency is developed differs depending on animal species. For example, in the case of mice, male germ cells will be deficient. Specifically, hybrid embryos obtained by reciprocal crossing or in vitro fertilization using C57BL/6 experimental mice and Mus spretus which is heterogenous mice from C57BL/6 result in male sterility (Matsuda Y et al., PNAS 88, 4850-4854 (1991)). Furthermore, embryos of c-kit mutant mice, for example, embryos obtained by reciprocal crossing or in vitro fertilization using Wv/+ mice and W/+ mice (Wv/W or W/Wv) cannot form both male and female germ cells (sterile/sterility). Thus, through the use of this genetic factor, mice lacking either a male or a female germ cell can be produced (Kussell ES, Adv Genet 20, 357-459 (1979)). In particular, when Wv/W or W/Wv embryos are used, female germ-line chimaeras can be produced using female ES cells. In addition, they can be produced as nuclear transferred embryos or transgenic embryos.

Since all the germ cells of the chimeric animal produced by the present invention are derived from ES cells, a male chimeric animal is subjected to crossing, artificial insemination, in vitro fertilization, or intracytoplasmic sperm injection, so that male and female heterozygotes having one chromosome of a chromosome pair of the ES cells can be obtained. By crossing the thus obtained male and female heterozygotes, homozygotes can be easily obtained.

Furthermore, male and female heterozygotes having one chromosome of a chromosome pair of ES cells can be obtained by subjecting a female chimeric animal produced from the ES cells subjected to female gene alteration to crossing, artificial insemination, in vitro fertilization, or intracytoplasmic sperm injection. The present invention provides methods described below and chimeric animals produced by such methods.
(1) A method for producing a germ-line chimeric animal wherein germ cells are derived from totipotent cells, which uses an early embryo incapable of forming germ cells and the totipotent cells.
(2) The production method of (1) above, wherein the early embryo incapable of forming germ cells is an embryo, a nuclear transferred embryo, or a transgenic embryo obtained by inter-specific crossing.
(3) The production method of (1) or (2) above, wherein the totipotent cells are embryonic stem cells (ES cells) or embryonic germ cells (EG cells).
(4) The production method of (1) or (2) above, wherein the totipotent cells are a nuclear transferred embryos, or embryonic stem cells (ES cells) or embryonic germ cells (EG cells) derived from a nuclear transferred embryos.
(5) The production method of any one of (1) to (4) above, wherein the animal is a mouse.
(6) The production method of (5) above, wherein the early embryo incapable of forming germ cells is an embryo obtained by reciprocal crossing or in vitro fertilization using an experimental mouse (Mus musculus domesticus) and a Mus spretus mouse which is in a sibling and heterogenous relationship therewith.
(7) The production method of any one of (1) to (5) above, wherein the early embryo incapable of forming germ cells is an embryo produced by c-kit mutation.
(8) A chimeric animal, which can be produced by the production method of any one of (1) to (7) above, and wherein germ cells are derived from introduced totipotent cells.
(9) A male chimeric animal, which can be produced by the production method of any one of (1) to (7) above, and wherein germ cells are derived from introduced totipotent cells.
(10) A female chimeric animal, which can be produced by the production method of any one of (1) to (7) above, and wherein germ cells are derived from introduced totipotent cells.
(11) The chimeric animal of any one of (8) to (10) above, wherein the animal is a mouse.
(12) A method for producing a heterozygous animal wherein one chromosome of a chromosome pair is derived from totipotent cells, which comprises:
   crossing a male chimeric animal wherein germ cells are derived from introduced totipotent cells with a female animal of a desired strain, or fertilizing an oocyte of a desired strain with a sperm cell obtained from the male chimeric animal by in vitro fertilization or intracytoplasmic sperm injection to obtain a embryo; and
   transferring the thus obtained embryo into a recipient animal so as to cause ontogeny.
(13) A method for producing a heterozygous animal wherein one chromosome of a chromosome pair is derived from totipotent cells, which comprises:
   crossing a female chimeric animal wherein germ cells are derived from introduced totipotent cells with a male animal of a desired strain, or fertilizing an oocyte obtained from the female chimeric animal with a sperm cell of a desired strain by in vitro fertilization or intracytoplasmic sperm injection to obtain an embryo; and
   transferring the thus obtained embryo into a recipient animal so as to cause ontogeny.
(14) A method for producing a homozygous animal, wherein the homozygous animal is obtained by carrying out crossing, artificial insemination, in vitro fertilization, or intracytoplasmic sperm injection using male and female chimeric animals obtained by (9) and (10) above.
(15) The production method of any one of (12) to (14) above, wherein the animal is a mouse.
(16) A heterozygous animal, which can be produced by any one of the methods of (12) to (14) above, and wherein one chromosome of a chromosome pair is derived from totipotent cells.
(17) A homozygous animal, which can be produced by any one of the methods of (12) to (14) above, and is obtained by crossing a female heterozygous animal with a male heterozygous animal wherein one chromosome of a chromosome pair is derived from totipotent cells.
(18) The animal of (16) or (17) above, wherein the animal is a mouse.

### [BEST MODE OF CARRYING OUT THE INVENTION]

The chimeric animal of the present invention is produced by injecting totipotent cells such as ES cells into an early embryo incapable of genetically forming germ cells. All the germ cells of the obtained chimeric animal are derived from the totipotent cells injected, such as ES cells when ES cells are used. In the following explanation, ES cells are illustrated as an example of totipotent cells. However, totipotent cells used herein are not limited to ES cells. All the sperm cells or oocytes of the obtained chimeric animal are derived from ES cells. Furthermore, chromosomes derived from ES cells are always transmitted to pups, and one chromosome of a chromosome pair is a heterozygote that is a gene of the ES cells.

An early embryo incapable of forming germ cells, such as an embryo, can be produced utilizing hypoplasia of a germ cell that takes place because of a genetic factor. To do this, 2 types of methods can be proposed. Specifically, since inter-specific F1 hybrid male mice show sterility, an embryo resulting from inter-specific crossing is obtained by naturally mating an experimental mouse (Mus musculus domesticus) with a mouse that is in a sibling and heterogenous relationship therewith. At this time, it is preferable to obtain oocytes from female experimental mice that have excellent proliferation ability. Specifically, it is preferable to obtain embryos resulting from inter-specific crossing by naturally mating female experimental mice (Mus musculus domesticus) with male mice that are in a sibling and heterogenous relationship therewith. In particular, a greater number of embryos can be obtained by subjecting 3-week- to 4-week-old young experimental female mice to hormone treatment so as to induce superovulation, followed by crossing.

Furthermore, inter-specific hybrid embryos obtained by carrying out in vitro fertilization between oocytes collected from superovulation-induced young female experimental mice and sperm cells of heterogenous mice can be efficiently obtained in large numbers. C57BL/6 mice and the like can be used as experimental mice.

In the meantime, as heterogenous mice for crossing, Mus spretus, Mus caroli, Mus pahari, and the like can be used. Mus spretus, for which a relatively large amount of information on in vitro fertilization has been accumulated, is particularly preferred. A combination of C57BL/6 female mice and Mus spretus male mice is particularly preferred, because inter-specific hybrid embryos can be stably obtained from this combination.

Furthermore, as a second method, embryos of c-kit mutant mice (Wv/W or W/Wv), for example, embryos obtained by reciprocal crossing or in vitro fertilization using Wv/+ mice and W/+ mice, can be produced utilizing the fact that hypoplasia of germ cells takes place. Particularly when Wv/W or W/Wv embryos are used, female germ-line chimeras can be produced using female ES cells.

All the thus obtained embryos are embryos that genetically form no germ cells. As a result, in the germ cells of a chimeric mouse obtained by injecting ES cells into this embryo, all the sperm cells or oocytes formed are derived from the ES cells.

The sperm cells (germ cells) of the thus obtained male chimeric mice are derived from the ES cells, and pups obtained by crossing the chimeric mouse with an experimental female mouse are heterozygous animals having one chromosome of a chromosome pair of the ES cells. The thus obtained chimeric mice are germ-line chimeric mice derived from the ES cells. Moreover, when the number of sperm cells of the obtained chimeric mouse is low, or the sperm activity is low, fertilization cannot be achieved by natural mating. However, even in such a case, pups can be obtained by collecting sperm cells and using technology such as intracytoplasmic sperm injection (ICSI). On the other hand, when Wv/W embryos are used, female germ-line chimeric animals can be produced using female ES cells. This is advantageous when a gene-altered animal wherein mitochondrial alteration or the like is conducted is produced.

As described above, germ-line chimeric animals can be efficiently produced according to the present invention. By the application of the present invention, it is predicted that gene-altered mice such as knockout mice can be produced from inbred-mouse-derived ES cells, which has hardly ever succeeded. Moreover, also from ES cells from which germ-line chimeras have never been obtained, gene-altered mice may be produced. In addition to ES cells, if cells are totipotent cells, application of the present invention may be possible.

Mice are used in the above explanation; however, this manipulation can be applied to all mammals. For example, in the case of domestic animals, efficient production of germ-line chimeric animals is made possible by producing clone embryos from somatic cells or gene-altered somatic cells by nuclear transfer, and then applying the method of the present invention. Furthermore, the method of the present invention can also be applied to experimental animals such as birds, reptiles, amphibians, and fish. In such cases, aneuploid embryos can be used as sterile embryos. The method of the present invention can also be applied to wild animals.

Moreover, male and female heterozygous animals wherein one chromosome of a chromosome pair is derived from ES cells can be obtained from pup of the next generation of the chimeric animals obtained by the present invention wherein all the germ cells are derived from the ES cells. Furthermore, homozygous animals (homo-type gene-altered animals) can be produced by crossing the thus obtained female heterozygous animal with the male heterozygous animal.

### [EXAMPLES]

The present invention will be further described specifically by referring to examples. However, these examples are simply intended to provide illustrations, and the present invention is not limited by these examples.

### Example 1

### Preparation of embryo to be used in chimera production

### (1) Obtainment of embryo incapable of forming male germ cells

Pregnant mare's serum gonadotropin (PMSG) was administered at a rate of 5 IU/0.05 ml/mouse to the abdominal cavities of 3.5-week-old C57BL/6 female mice (B6-mtSPE: C57BL/6 mice wherein cytoplasmic mitochondria had been substituted with those of wild-type-derived inbred Mus spretus mice by backcrossing) having Mus spretus-type mitochondria. 48 hours later, human chorionic gonadotropin (hCG) was administered at a rate of 5 IU/0.05 ml/mouse so as to conduct superovulation induction treatment. Next, female mice subjected to superovulation induction treatment were naturally mated with sexually mature (6-week-old or older) male wild-type-derived inbred Mus spretus mice by allowing them to live together. On day 3.5 after the confirmation of mating, uteri of the female mice were extracted. The uteri were perfused with a M2 medium (94.7 mM NaCl, 4.78 mM KC1, 1.71 mM CaCl₂, 1.19 mM KH₂PO₄, 1.19 mM MgSO₄, 4.00 mM NaHCO₃, 21.0 mM HEPES, 23.3 mM sodium lactate, 0.33 mM sodium pyruvate, 1 g/L glucose, 4 g/L BSA, 100 IU/mL penicillin, 50 µg/mL streptomycin), thereby collecting embryos. The embryos were washed with the same medium, thereby preparing sibling-heterogenous embryos (blastocyst-stage embryos).

In the meantime, when sibling-heterogenous embryos were prepared by in vitro fertilization, and C57BL/6 female mice subjected to superovulation induction treatment according to the above method (16 hours after administration of hCG) were euthanized by vertebral dislocation. Oviduct was extracted from the mice, and then allowed to come into contact with mineral oil portions of droplets of mTYH medium coated with mineral oil (199.37 mM NaCl, 4.78 mM KC1, 1.71 mM CaCl₂, 1.19 mM KH₂PO₄, 1.19 mM MgSO₄, 25.07 mM NaHCO₃, 1.00 mM sodium pyruvate, 4 g/L BSA, 100 IU/mL penicillin, 50 µg/mL streptomycin). Next, the oviduct was broken using two 27G injection needles under a stereoscopic microscope, and then a cumulus cell group including oocytes was introduced into the mTYH medium droplets. In addition, the cell group was carefully introduced while preventing the oviducts from entering within the medium so as to prevent contamination with blood or tissue. Sperm cells to be pre-cultured were prepared by euthanizing each male Mus spretus mouse by vertebral dislocation, extracting the cauda epididymis, wiping off the blood attached to the cauda epididymis using Kimwipe, making a cut using a 23G injection needle in the thickest seminal duct while holding the duct with the fingers, and then scraping out sperm mass coming from the duct using an injection needle. The sperm cells were then introduced into 0.3 ml of a mTYH medium (liquid) coated with mineral oil or a mTYH medium (199.37 mM NaCl, 4.78 mM KC1, 1.71 mM CaCl₂, 1.19 mM KH₂PO₄, 1.19 mM MgSO₄, 25.07 mM NaHCO₃, 1.00 mM sodium pyruvate, 1 g/L glucose, 4 g/L BSA, 100 IU/mL penicillin, and 50 µg/mL streptomycin), and then cultured for 2 to 8 hours in a CO₂-incubator. The thus precultured sperm cells were inseminated into a medium for fertilization (mTYH medium) containing previously prepared oocytes at a final sperm concentration of 1 to 5×10⁵ sperm cells/mL. Oocytes subjected to insemination were cultured within a CO₂-incubator for 8 to 10 hours. Next, the oocytes were washed with an M16 medium for development supplemented with 0.1 mM EDTA (94.59 mM NaCl, 4.78 mM KC1, 1.71 mM CaCl₂, 1.19 mM KH₂PO₄, 1.19 mM MgSO₄, 25.07 mM NaHCO₃, 23.28 mM sodium lactate, 0.33 mM sodium pyruvate, 1 g/L glucose, 4 g/L BSA, 100 IU/mL penicillin, and 50 µg/mL streptomycin), and then transferred into the same medium. After fertilized eggs were confirmed under a stereoscopic microscope, the eggs were cultured within a CO₂-incubator for 96 hours, thereby preparing blastocyst-stage embryos.

### (2) Obtainment of embryo incapable of forming both male and female germ cells

Crossing or in vitro fertilization was conducted using male and female W-strain heterozygous mice having c-kit mutation involved in germ cell formation, and then embryos of Wv/W or W/Wv homozygotes were produced according to the. method described in (1), thereby preparing blastocyst-stage embryos.

### Example 2

### Production of chimeric mouse

Into the blastocyst-stage embryos (Mus spretus-type mitochondrial DNA) that had been prepared by in vitro fertilization using oocytes of inbred female C57BL/6 mice (B6-mtSPE) having a Mus spretus-type mitochondrial DNA and wild-type-derived inbred Mus spretus sperm cells described in Example 1, inbred C57BL/6-derived ES cells (Mus musculus domesticus-type mitochondrial DNA) were injected according to the standard methods. 48 blastocyst-stage embryos were used. 5 ES cells were injected into one embryo, thereby obtaining 48 embryos subjected to injection. 16 embryos subjected to injection were implanted into the uteri of three 6-week-old recipient female CD-1 mice on day 2 after crossing with 8-week-old vasoligated male mice, thereby obtaining 3 chimeric pup.

### Example 3

### Chimeric mouse germ-line lineage

Male chimeric mice obtained in Example 2 were grown to reach their sexual maturity. 3 types of mice were selected in terms of coat color (low mosaic, medium mosaic, and black), and then sperm cells were collected from the caudal epididymis. Germ cell lineage was examined by carrying out PCR analysis on the thus obtained sperm mitochondrial DNAs. When sperm cells were derived from the ES cells, Mus musculus domesticus-type mitochondrial DNAs were detected, and when derived from the fertilized egg, Mus spretus-type mitochondrial DNAs were detected. As a result of analyses made by the nested-PCR method (Kaneda H et al., PNAS 92, 4542-4546 (1995)), all the sperm cells obtained from these mice were derived from the ES cells. Furthermore, no sperm cells could be confirmed in inter-specific F1 hybrid male mice that had been obtained by in vitro fertilization using the oocytes of C57BL/6 mice (B6-mtSPE) and Mus spretus sperm cells, and no pup could be obtained by crossing with C57BL/6 mice. Thus, it was confirmed that inter-specific F1 hybrid male mice were unable to form germ cells.

### [Industrial Applicability]

Germ cells produced by chimeric animals obtained according to the present invention are derived from injected ES cells. Hence, male and female heterozygous animals wherein one chromosome of a chromosome pair is derived from ES cells can be obtained by crossing the chimeric animal with an animal of a desired strain, or carrying out in vitro fertilization using a sperm cell obtained from the chimeric animal and an oocyte of a desired strain, and then implanting the thus obtained fertilized egg into a recipient animal so as to obtain pup. A homozygous animal can be produced by crossing the thus obtained female heterozygous animal with the male heterozygous animal. In general, as ES cells used herein, ES cells that have been previously subjected to desired gene alteration by homologous recombination or the like can be used. Thus, gene-altered homozygous animals can be easily obtained. Furthermore, when embryos of c-kit mutant mice, such as Wv/W or W/Wv embryos obtained by reciprocal crossing or in vitro fertilization using Wv/+ mice and W/+ mice are used, it is impossible to form not only male, but also female germ cells. Hence, irrespective of whether ES cells are male or female, the injected ES cells contribute to all the germ cells of the obtained chimeric mice, so that germ-line chimeric animals can be produced with either female or male ES cells (ES cells used herein are not limited to male ES cells).

Furthermore, all the germ cells of a chimeric animal produced according to the present invention are derived from ES cells. Thus, male and female heterozygotes having one chromosome of a chromosome pair of ES cells can be obtained by crossing with male chimeric animals or in vitro fertilization using the same. The thus obtained male and female heterozygotes can be crossed with each other to be able to easily obtain homozygotes, so that they are extremely useful for gene function analyses. Furthermore, male and female heterozygotes having one chromosome of a chromosome pair of ES cells can be obtained by crossing or artificial insemination using female chimeric animals produced by establishing gene-altered female ES cells. Homozygotes can be easily obtained similarly by crossing the thus obtained female heterozygote with the male heterozygote. Furthermore, it is thought that homozygotes can also be' obtained by crossing female chimeric animals with male chimeric animals. Gene-altered animals obtained by the above methods are extremely useful in gene function analyses.

As described above, it is made possible to conduct such manipulations conveniently, making it possible to efficiently produce a germ-line chimeric animal, of which production has conventionally been inefficient.

## Claims

1. A method for producing a germ-line chimeric animal wherein germ cells are derived from totipotent cells, which uses an early embryo incapable of forming germ cells and the totipotent cells.

2. The production method of claim 1, wherein the early embryo incapable of forming germ cells is an embryo, a nuclear transferred embryo, or a transgenic embryo obtained by inter-specific crossing.

3. The production method of claims I or 2, wherein the totipotent cells are embryonic stem cells (ES cells) or embryonic germ cells (EG cells).

4. The production method of claim 1 or 2, wherein the totipotent cells are nuclear transferred embryos, or embryonic stem cells (ES cells) or embryonic germ cells (EG cells) derived from nuclear transferred embryos.

5. The production method of any one of claims 1 to 4, wherein the animal is a mouse.

6. The production method of claim 5, wherein the early embryo incapable of forming germ cells is an embryo obtained by reciprocal crossing or in vitro fertilization using an experimental mouse (Mus musculus domesticus) and a Mus spretus mouse which is in a sibling and heterogenous relationship therewith.

7. The production method of any one of claims 1 to 5, wherein the early embryo incapable of forming germ cells is an embryo produced by c-kit mutation.

8. A chimeric animal, which can be produced by the production method of any one of claims 1 to 7, and wherein germ cells are derived from introduced totipotent cells.

9. A male chimeric animal, which can be produced by the production method of any one of claims 1 to 7, and wherein germ cells are derived from introduced totipotent cells.

10. A female chimeric animal, which can be produced by the production method of any one of claims 1 to 7, and wherein germ cells are derived from introduced totipotent cells.

11. The chimeric animal of any one of claims 8 to 10, wherein the animal is a mouse.

12. A method for producing a heterozygous animal wherein one chromosome of a chromosome pair is derived from totipotent cells, which comprises:
crossing a male chimeric animal wherein germ cells are derived from introduced totipotent cells with a female animal of a desired strain, or fertilizing an oocyte of a desired strain with a sperm cell obtained from the male chimeric animal by in vitro fertilization or intracytoplasmic sperm injection to obtain an embryo; and
transferring the thus obtained embryo into a recipient animal so as to cause ontogeny.

13. A method for producing a heterozygous animal wherein one chromosome of a chromosome pair is derived from totipotent cells, which comprises:
crossing a female chimeric animal wherein germ cells are derived from introduced totipotent cells with a male animal of a desired strain, or fertilizing an oocyte obtained from the female chimeric animal with a sperm cell of a desired strain by in vitro fertilization or intracytoplasmic sperm injection to obtain an embryo; and
transferring the thus obtained embryo into a recipient animal so as to cause ontogeny.

14. A method for producing a homozygous animal, wherein the homozygous animal is obtained by carrying out crossing, artificial insemination, in vitro fertilization, or intracytoplasmic sperm injection using male and female chimeric animals obtained by claims 9 and 10.

15. The production method of any one of claims 12 to 14, wherein the animal is a mouse.

16. A heterozygous animal, which can be produced by any one of the methods of claims 12 to 14, and wherein one chromosome of a chromosome pair is derived from totipotent cells.

17. A homozygous animal, which can be produced by any one of the methods of claims 12 to 14, and is obtained by crossing a female heterozygous animal with a male heterozygous animal wherein one chromosome of a chromosome pair is derived from totipotent cells.

18. The animal of claim 16 or 17, wherein the animal is a mouse.
